# FASCICULE DE BREVET EUROPEEN

(11) **EP 1 201 219 B1**
(45) Date de publication et mention de la délivrance du brevet: **14.12.2005**
(21) Numéro de dépôt: 01402673.6
(22) Date de dépôt: 17.10.2001
(51) Int. Cl.: A61K 7/00, A61K 7/48, A61K 9/50

(54) **Utilisation de microcapsules thermostabilisatrices pour améliorer l'activité ou la pénétration de principes actifs cosmétiques ou pharmaceutiques**
Verwendung von thermostabilisierenden Mikrokapseln zur Verbesserung der Aktivität oder der Eindringung von kosmetischen oder pharmazeutischen Wirkstoffen
Use of thermostabilising microcapsules for improving the activity or the penetration of cosmetic or pharmaceutical active agents

(30) Priorité: 27.10.2000 FR 0013794
(43) Date de publication de la demande: 02.05.2002
(73) Titulaire: L'OREAL, 75008 Paris (FR)
(72) Inventeur: Afriat, Isabelle, 75003 Paris (FR); Biatry, Bruno, 94300 Vincennes (FR)
(74) Mandataire: Casalonga, Axel

(56) Documents cités:
- EP-A- 0 303 461
- EP-A- 0 306 202
- WO-A-00/56940
- WO-A-93/24241
- WO-A-94/23688
- FR-A- 2 732 240
- US-A- 5 499 460

## Description

La présente invention concerne l'utilisation de microcapsules thermostabilisatrices pour améliorer l'activité ou la pénétration de principes actifs cosmétiques ou pharmaceutiques, des compositions topiques contenant des principes actifs cosmétiques ou pharmaceutiques associés à de telles microcapsules, ainsi que des procédés de traitement cosmétique de la peau utilisant ces compositions topiques.

Dans le domaine cosmétique ou pharmaceutique il existe de nombreuses applications nécessitant un apport de chaleur.
En effet, la production locale de chaleur peut par exemple favoriser le nettoyage de la peau par un effet de dilatation des pores ou renforcer l'action d'un produit, tel qu'une crème amincissante, par activation de la microcirculation sanguine ou par amélioration de la pénétration cutanée du principe actif appliqué.

Cette chaleur peut être apportée par exemple par un massage des zones à traiter ce qui nécessite cependant un effort parfois prolongé que l'utilisateur n'est pas toujours prêt à fournir.
On utilise également souvent à cet effet la chaleur de dilution de polyéthylèneglycols concentrés. Cette approche présente cependant l'inconvénient que le contact direct entre le produit chimique (polyéthylèneglycol) et la peau peut conduire à des irritations cutanées.

Il existe par conséquent un besoin de compositions cosmétiques ou pharmaceutiques permettant un apport local de chaleur mais qui n'impliquent pas un contact direct du produit chauffant avec la peau et ne nécessitent pas un effort de massage.

La demanderesse a découvert de manière surprenante qu'il était possible de résoudre le problème indiqué ci-dessus grâce à l'utilisation de microcapsules particulières, décrites plus en détail ci-dessous, ayant des capacités d'absorption et de restitution de chaleur.

Les microcapsules en question contiennent, dans une enveloppe étanche, des composés partiellement ou complètement cristallins qui, lorsqu'on les porte à une température proche de leur température de fusion, absorbent une importante quantité de chaleur, appelée chaleur latente de fusion. L'absorption de cette chaleur latente de fusion se traduit par une stabilité de la température du composé malgré l'apport d'énergie thermique. Cet effet est semblable à un effet "tampon" thermique et permet de thermostater, pendant un certain temps et dans un intervalle de température proche de la température de fusion du composé, l'environnement direct des microcapsules malgré une variation de la température extérieure.
La capacité d'absorption de chaleur décrite ci-dessus va de paire avec la possibilité, mise à profit dans la présente invention, de restituer l'énergie absorbée sous forme de chaleur latente de cristallisation. En effet, lorsqu'on abaisse la température d'un tel composé à l'état fondu en dessous de son point de fusion, on constatera localement et pendant un certain temps une stabilisation de la température malgré le refroidissement environnant. Les microcapsules contenant le composé à l'état fondu constituent ainsi une réserve d'énergie thermique.

Les microcapsules ayant les capacités d'absorption et de restitution de chaleur réversibles décrites ci-dessus seront appelées par la suite "microcapsules thermostabilisatrices".

La présente invention a par conséquent pour objet des compositions topiques comprenant, dans un support physiologiquement acceptable, l'association d'au moins un principe actif cosmétique ou pharmaceutique et de microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C.

L'invention a également pour objet une composition topique comprenant, dans un support physiologiquement acceptable, l'association d'au moins un principe actif pharmaceutique et de microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, en tant que médicament.

L'invention a en outre pour objet des procédés de traitement cosmétique comprenant l'application séquentielle ou simultanée, d'au moins un principe actif cosmétique et de microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, préalablement chauffées jusqu'à une température supérieure au point de fusion du composé cristallin encapsulé.

Enfin, l'invention a pour objet l'utilisation des microcapsules décrites ci-dessus pour la fabrication d'un produit destiné à améliorer l'activité et/ou la pénétration cutanée d'un principe actif pharmaceutique appliqué simultanément ou préalablement.

L'efficacité des compositions cosmétiques de la présente invention dépend directement de la capacité d'absorption thermique des composés cristallins encapsulés et de leur point de fusion.

La capacité d'absorption thermique des microcapsules utilisées est directement proportionnelle à l'enthalpie de fusion du composé cristallin encapsulé. Cette enthalpie de fusion est mesurée par analyse enthalpique différentielle (en anglais : *Differential Scanning Calorimetry*).
L'enthalpie de fusion d'un composé est la quantité d'énergie nécessaire pour transformer un échantillon partiellement ou totalement cristallin en un échantillon totalement amorphe. Le thermogramme ΔCp = f(T), dans lequel ΔCp représente la différence de capacité thermique de l'échantillon par rapport à un échantillon de référence ne subissant aucune transition thermique dans le domaine étudié, présente donc un signal endothermique dont l'aire est proportionnelle à l'enthalpie de fusion (ΔH_{f}) de l'échantillon.

Le terme "composé cristallin" tel qu'il est utilisé dans la présente demande englobe les composés partiellement ou totalement cristallins. Le taux de cristallinité des composés utilisés n'est pas déterminant dans la mesure où le composé présente l'enthalpie de fusion cristalline requise pour l'utilisation envisagée.

Comme indiqué, les microcapsules utilisées dans les compositions cosmétiques de la présente invention contiennent des composés cristallins ayant une enthalpie de fusion comprise entre 75 et 330 kJ/kg, de préférence entre 100 kJ/kg et 300 kJ/kg et idéalement entre 150 et 280 kJ/kg.

Le composé cristallin encapsulé doit avoir une température de fusion (= température de cristallisation) supérieure ou égale à 30 °C qui est une température proche de celle de la peau.
Il s'agit là d'une caractéristique essentielle de la présente invention car en effet, pour obtenir l'effet de stockage de chaleur recherché, la cristallisation du composé cristallin encapsulé doit avoir lieu à une température proche de celle de la peau et de préférence supérieure ou égale à celle-ci.
Bien entendu, le composé cristallin ne doit pas avoir un point de fusion trop élevé correspondant à une température qui provoque une sensation de chaleur excessive désagréable, voire même des brûlures.
L'intervalle de température de fusion des composés cristallins encapsulés dans les microcapsules thermostabilisatrices utilisées dans la présente invention va notamment de 30 à 45 °C et de préférence de 32 °C à 40 °C.

On peut citer à titre d'exemples de composés cristallins encapsulés appropriés pour la présente invention :
- les hydrocarbures aliphatique, de préférence à chaîne linéaire, contenant de 13 à 28 atomes de carbone, de préférence de 19 à 22 atomes de carbone,
- les hydrocarbures aromatiques,
- les acides gras saturés ou insaturés en C₉₋₂₄ et notamment l'acide caprique, l'acide laurique et l'acide élaïdique,
- les alcools gras saturés, linéaires ou ramifiés en C₁₄₋₃₆, et notamment l'alcool myristylique ou l'hexadécyl-2-éicosanol,
- les esters des acides gras en C₁₀₋₂₂ tels que le stéarate de benzoyle, le cinnamate de méthyle, le palmitate de méthyle, le béhénate d'isostéaryle, le tétralaurate de di-triméthylolpropane vendu sous la dénomination HEST® 2T-4L par la société HETEREN, et le tétrahydroxystéarate de di-triméthylolpropane vendu sous la dénomination HEST® 2T-5E-4HS par la société HETEREN,
- les sels minéraux contenant une importante fraction d'eau de cristallisation, tels que l'hexahydrate de chlorure de calcium, le décahydrate de sulfate de sodium, le dodécahydrate d'hydrogénophosphate de sodium, le pentahydrate de thiosulfate de sodium et l'hexahydrate de nitrate de nickel,
- les triglycérides d'acides gras en C₁₂₋₁₈,
- certaines cires de silicone telles que les polydiméthylsiloxanes à groupements terminaux béhénoxy ou stéaroxy (INCI : behenoxydimethicone et stearoxydimethicone), les polyméthylstéaryloxy-diméthylsiloxane (INCI : stearic ester dimethicone), les polyméthylstéaryl-diméthylsiloxane (INCI : stéaryldimethicone), des copolymères à motifs méthacrylate de stéaryle avec greffons polydiméthylsiloxane, les polyméthyltrifluorométhylalkyl-diméthylsiloxane (INCI : trifluorométhyl(C₁₋₄ alkyl)-diméthicone.
- les dérivés de la cire d'abeille, comme la cire d'abeille estérifiée par un diméthiconol vendue sous la dénomination ULTRABEE® par la société JW HANSON.

Ces composés peuvent être utilisés seuls ou sous forme de mélange de deux ou plusieurs d'entre-eux.

On peut également citer les polymères thermofusibles au moins partiellement cristallins présentant un point de fusion cristalline appropriée.
De tels polymères sont par exemple les homopolymères et copolymères oléfiniques, y compris les cires polyoléfiniques, tels que les homopolymères d'éthylène, les copolymères d'éthylène et de propylène, les copolymères d'éthylène et d'octène, les copolymères d'éthylène et de butène et les copolymères d'éthylène et d'acétate de vinyle.
On peut également citer les poly(alkylène oxyde), les polyesters d'alkyle, les poly(e-caprolactones), les polyamides, en particulier ceux résultant de la polycondensation de dimère d'acides gras, et les copolymères d'oléfines fluorées

Un autre groupe de polymères cristallins utilisables est formé par les polymères à chaînes latérales cristallisables décrits dans *J. Polymer Sci. : Macromol. Rev. 8:117-253 (1974).* Il s'agit de polymères ou copolymères vinyliques et/ou acryliques contenant une fraction importante, généralement au moins égale à 50 % en poids, de motifs copolymérisés comportant de longues chaînes latérales aliphatiques linéaires cristallisables, ou des chaînes latérales fluorées ou perfluorées cristallisables. Le brevet US 5 156 911 décrit l'utilisation de tels polymères à chaînes latérales cristallisables dans des assemblages adhésifs dont les propriétés adhésives varient en fonction de la température.

Les composés cristallins encapsulés préférés pour la présente invention sont les hydrocarbures aliphatiques à chaîne linéaire contenant de 19 à 28 atomes de carbone, de préférence de 20 à 23 atomes de carbone, à savoir le n-nonadécane, le n-eicosane, le n-henéicosane, le n-docosane et le n-tricosane.

Selon la présente invention, les composés cristallins sont encapsulés dans une enveloppe étanche.
Cette encapsulation est une condition essentielle pour la réversibilité des processus de fusion/cristallisation. En effet, en l'absence d'une enveloppe étanche, le composé fondu diffuserait à travers la membrane et dans la composition cosmétique et l'effet thermique lié à sa cristallisation se trouverait fortement réduit, voire même supprimé.
Pour cette même raison, l'enveloppe doit être suffisamment solide pour résister aux forces de cisaillement lors de l'application de la composition les contenant.

Le matériau constituant la paroi des microcapsules peut être choisi parmi tous les matériaux classiquement utilisés dans le domaine de la microencapsulation. Ce matériau peut être amorphe, cristallin ou semi-cristallin. Lorsqu'il est cristallin ou semi-cristallin, il doit avoir un point de fusion supérieur à celui des composés cristallins encapsulés. Par ailleurs, ce matériau doit être suffisamment élastique pour résister aux variations de volume du composé cristallin lors de la transition de phase. De plus il doit être inerte vis-à-vis des substances encapsulées et des composés de la formulation cosmétique ou pharmaceutique avec laquelle il sera en contact.

Selon le procédé choisi, on pourra utiliser comme matériaux des polymères tels que les polyamides, les polyuréthanes, les polyurées, les polyesters, les polycyanoacrylates, les résines urée-formaldéhyde ou mélamine-formaldéhyde, et les systèmes gélatine/gomme arabique.

Les microcapsules peuvent être préparées selon des procédés bien connus décrits par exemple dans l'ouvrage intitulé *"Microencapsulation, Methods and Industrial Applications",* Directeur de la publication S. Benita, Marcel Dekker (1996). On peut citer à titre d'exemple la polymérisation ou polycondensation interfaciale, la coacervation, l'atomisation, l'extrusion centrifuge ou la microencapsulation sur disques rotatifs.

Les microcapsules thermostabilisatrices sont connues et commercialisées par exemple sous la dénomination Thermasorb® par la société Frisby Technologies Inc. ou sous les références 9850K et 9850Q par la société 3M.
Ces microcapsules se présentent sous forme d'une poudre fine, fluide et non filmogène. Leur utilisation est connue par exemple dans le domaine des vêtements et chaussures isothermes, dans des systèmes de refroidissement en microélectronique et dans le domaine de l'emballage.

On peut également utiliser comme composé constituant la paroi des microparticules un matériau non polymérique. On peut utiliser par exemple des microcapsules à base de silice précipitée, amorphe, hydratée ou rendue hydrophobe, proposées par la société PHASE CHANGE LABO-RATORIES sous la dénomination AcuTemp® .

La taille supérieure des microcapsules à enveloppe étanche utilisées dans la présente invention est de préférence limitée, pour des raisons évidentes de visibilité, à quelques dizaines ou centaines de micromètres. On préfère généralement utiliser des microcapsules ayant un diamètre moyen compris entre 0,01 et 100 micromètres, mieux encore entre 0,05 et 50 micromètres.

La proportion des microcapsules dans les compositions topiques de la présente invention peut varier entre de larges limites qui dépendent de la formulation et de l'application envisagée.
Les compositions topiques de la présente invention contiennent généralement de 0,1 % à 95 % en poids et de préférence de 5 % à 90 % en poids de microcapsules thermostabilisatrices, rapporté à la composition topique finale.

Les principes actifs cosmétiques ou pharmaceutiques utilisables dans les compositions topiques de la présente invention sont tous ceux dont l'activité ou la pénétration est susceptible d'être augmentée par un apport local de chaleur.
On peut citer à titre d'exemples de principes cosmétiques ou pharmaceutiques utilisables selon la présente invention
- les agents anti-rides tels que le rétinol et ses dérivés (acétate, palmitate, propionate), les rétinoïdes, l'acide n-octanoylsalicylique et les hydroxyacides,
- les agents antibactériens et/ou antifongiques tels que la chlorhexidine, l'hexétidine, l'hénamidine et le chlorure de benzalkonium,
- les agents anti-acnéiques tels que le triclosan, l'acide azélaïque, le peroxde de benzoyle et l'acide salicylique,
- les agents anti-radicaux libres et/ou détoxifiants tels que l'acide ascorbique et les dérivés de celui-ci comme l'ascorbylphosphate de magnésium, les protéines et les enzymes, par exemple la superoxyde dismutase (SOD), les peroxydases telles que la lactoperoxydase et la lactoferrine, la catalase, les protéases telles que la subtillisine et la papaïne, les lipases, l'uricase, les peptides et leurs dérivés, l'ubiquinone et le cytochrome C,
- les agents kératolytiques tels que les α-hydroxyacides, les β-hydoxyacides et les α-cétoacides comme l'acide salicylique et ses dérivés,
- les accélérateurs de bronzage tels que les dérivés de tyrosine, les actifs dépigmentants tels que l'acide kojique, l'arbutine et les dérivés de ceux-ci,
- les colorants naturels extraits de végétaux comme la chlorophylline, ou extraits d'animaux comme le carmin de cochenille, ou le caramel,
- les actifs autobronzants tels que la dihydroxyacétone et les indoles,
- les liporégulateurs tels que la caféine et la théophylline,
- les agents hydratants tels que le sorbitol, le xylitol, l'urée et l'ADN végétal,
- les agents antipelliculaires tels que la piroctone olamine et les dérivés de pyridinethione,
- les agents anti-chute des cheveux tels que le minoxidil.

Les compositions topiques de la présente invention peuvent contenir en outre des adjuvants appropriés tels que des solvants, des conservateurs, des agents régulateurs de pH, des agents anti-oxydants, des agents séquestrants, des agents conservateurs, des pigments et colorants, des charges, des émollients, des agents anti-mousse, des corps gras tels que les huiles, les cires et les corps gras pâteux, des agents dispersants, des silicones telles que les huiles volatiles ou non, les gommes, les cires ou les pâteux, des parfums, des agents tensioactifs, des plastifiants, des polymères filmogènes solubles ou dispersibles et des polymères épaississants ou gélifiants.

Les compositions topiques de la présente invention se présentent par exemple sous forme de masque pour le visage ou les cheveux, de produit de massage, de composition amincissante, de crème hydratante, de crème anti-rides, de crème solaire, de composition de nettoyage, de lotion, de cataplasme, de produit relaxant ou de crème dépilatoire.

La présente invention a également pour objet des substrats solides insolubles, imprégnés avec une composition topique telle que décrite ci-dessus.

En effet, les compositions cosmétiques ou pharmaceutiques de la présente invention peuvent également servir à imprégner un substrat solide insoluble. Le substrat insoluble peut être choisi dans le groupe comprenant les matériaux textiles tissés ou non tissés, les mousses, les éponges, les ouates ou les billes. Il peut s'agir notamment d'un substrat textile non-tissé à base de fibres d'origine naturelle telles que les fibres de lin, de coton ou de soie, ou d'origine synthétique telles que les fibres de cellulose, de viscose, de polymères vinyliques, de polyesters comme le poly(téréphtalate d'éthylène), les polyoléfines comme le polyéthylène ou le polypropylène, les polyamides comme le Nylon® ou les polymères acryliques. Les matériaux non-tissés sont décrits par exemple dans "Nonwaven Binding Methods & Materials", de Riedel, Nonwoven World, 1987. Ces substrats sont obtenus selon des procédés connus dans le domaine de la technique de préparation des non-tissés.
Ce substrat peut comporter une ou plusieurs couches ayant des propriétés identiques ou différentes, et qui peuvent apporter par exemple des propriétés d'élasticité ou de douceur selon l'usage envisagé. Les substrats peuvent par exemple comporter deux parties ayant des propriétés d'élasticité différentes, comme ceux décrits dans la demande internationale WO99/13861 ou bien comporter une seule couche avec des densités différentes comme décrit dans le document WO 99/25318, ou encore comporter deux couches de texture différente comme les substrats décrits par exemple dans la demande internationale WO 98/18441.
Le substrat peut avoir n'importe quelle taille ou forme appropriée à l'application envisagée.
Il a généralement une surface comprise entre 0,005 m² et 0,1 m², de préférence entre 0,01 m² et 0,05 m². Il se présente de préférence sous la forme de lingettes rectangulaires ou de compresses rondes.
L'article final comportant le substrat et la composition d'imprégnation est généralement à l'état humide, avec un taux d'imprégnation, c'est-à-dire une quantité de composition rapportée au poids du substrat solide, compris entre 200 et 1000 %, de préférence entre 250 et 350 %.
Les techniques d'imprégnation des substrats sont bien connues dans le domaine technique et sont toutes applicables à la présente invention. Le plus souvent, la composition d'imprégnation est ajoutée au substrat par une ou plusieurs techniques telles que l'immersion, l'enduction ou la vaporisation.
Il est également possible de constituer un article (ou lingette) présenté à l'état sec soit en éliminant l'eau de la composition après son imprégnation sur le substrat, soit en déposant sur le substrat une composition sous forme sèche à l'état de poudre, granule ou film, par tous les moyens de réalisation connus tels que le soudage et le collage de multicouches par voie thermique ou par ultrasons. Dans ce dernier mode de réalisation, la composition est séchée par tout moyen connu, par exemple par atomisation, lyophilisation ou autre technique analogue.
On peut ainsi obtenir, selon l'utilisation envisagées, des lingettes humides ou des lingettes sèches. Les lingettes humides peuvent être utilisées telles quelles alors que les lingettes sèches sont mouillées avant utilisation.

L'invention a également pour objet des procédés de traitement cosmétique utilisant les microcapsules thermostabilisatrices décrites ci-dessus. Ces procédés ont en commun le chauffage des microcapsules jusqu'à une température supérieure à la température de fusion des composés cristallins encapsulés, l'application des microcapsules chauffées contenant le composé encapsulé à l'état fondu et le lent refroidissement de la ou des compositions contenant les microcapsules appliquées à chaud. Le principe actif cosmétique peut se trouver dans la composition contenant les microcapsules thermostabilisatrices et il est alors chauffé et appliqué en même temps que celles-ci, mais on peut tout aussi bien envisager une application séparée, préalable du principe actif, suivie de l'application d'une deuxième composition contenant les microcapsules thermostabilisatrices chauffées. Ce deuxième mode de réalisation est particulièrement intéressant pour des principes actifs thermosensibles ne supporttant pas une exposition prolongée à une température élevée.

Par conséquent, dans un premier mode de réalisation, le procédé de traitement cosmétique comprend les étapes suivantes consistant
- à chauffer une composition cosmétique contenant au moins un principe actif cosmétique et des microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, jusqu'à une température supérieure au point de fusion dudit composé cristallin,
- à appliquer la composition cosmétique sur la zone de la peau à traiter,
- à laisser la composition cosmétique refroidir au contact de la peau pendant un temps suffisant pour obtenir l'effet cosmétique recherché, et éventuellement,
- à éliminer la composition cosmétique.

Un autre mode de réalisation du procédé de traitement cosmétique de la présente invention comprend les étapes consistant
- à appliquer une composition cosmétique contenant au moins un principe actif cosmétique sur la zone de la peau à traiter,
- à chauffer une deuxième composition contenant, dans un support cosmétiquement acceptable, des microcapsule à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, jusqu'à une température supérieure au point de fusion dudit composé cristallin,
- à appliquer cette deuxième composition sur la zone de la peau à traiter, au dessus de la première composition cosmétique,
- à laisser les deux compositions au contact de la peau pendant un temps suffisant pour obtenir l'effet cosmétique recherché, et éventuellement
- à nettoyer la zone de la peau à traiter.

Un troisième mode de réalisation du procédé de traitement cosmétique de la présente invention comprend les étapes consistant
- à appliquer sur la zone du corps ou du visage à traiter une composition cosmétique contenant au moins un principe actif cosmétique et des microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C,
- à masser la zone enduite de manière à augmenter localement la température jusqu'à une valeur suffisante pour faire fondre le composé cristallin,
- à laisser la composition cosmétique agir et refroidir au contact de la peau pendant un temps suffisant pour obtenir l'effet cosmétique recherché, et éventuellement,
- à éliminer la composition cosmétique.

Ces procédés de traitement cosmétique tirent profit de la capacité de stockage thermique des microcapsules thermostabilisatrices décrites ci-dessus. En effet, lors du refroidissement de la ou des compositions appliquées sur la zone à traiter, le composé encapsulé fondu recristallisera en réstituant la chaleur latente de fusion absorbée lors du chauffage préalable des microcapsules. Cette prolongation de l'effet thermique augmentera la pénétration cutanée du principe actif et/ou son activité cosmétique.

### Exemple 1

### Gel de massage

| | | |
|---|---|---|
| A | Polymère carboxyvinylique (Sepigel® 305, SEPPIC) | 0,2 g |
| | Polymère carboxyvinylique (Pemulen® , GOODRICH) | 1,5 g |
| | Glycérol | 3 g |
| | Eau déminéralisée | 35 g |
| | Triéthanolamine | 0,5 g |
| | | |
| B | Huile de silicone | 5 g |
| | Squalane | 2 g |
| | Gomme de silicone | 1 g |
| | Parfum | 0,2 g |
| | Colorant | 0,02 g |
| | | |
| C | Alcool éthylique | 20 g |
| | Caféine | 3 g |
| | Eau déminéralisée | 18,58 g |
| | | |
| D | Microcapsules d'eisocane (9850 Q, 3M) | 10 g |

### Exemple 2

### Composition ne nettoyage

### Phase A

- Stéaryate de glycéryle/stéarate de PEG 100 (Arlacel® 165) 0,55 %
- Alcool cétylique 0,15 %
- Gomme de xanthane 0,1 %
- Palmitate d'isopropyle 3,8 %

### Phase B

- Eau qsp 100 %
- Conservateur qs

### Phase C

- Microcapsules thermostabilisatrices 10 %
On chauffe séparément les phases A et B jusqu'à une température comprise entre 75 et 80 °C, puis on ajoute la phase A dans la phase B sous agitation. On maintient l'agitation pendant 5 minutes. On ajoute la phase C sous faible agitation. On laisse refroidir jusqu'à la température ambiante sous faible agitation et on imprègne des lingettes avec l'émulsion obtenue.

### Exemple 3

### Lingettes amincissantes

On utilise une lotion préparée à partir des ingrédients suivants pour imprégner des lingettes :

| | |
|---|---|
| Caféine | 3 % |
| Triéthanolamine | 0,4 % |
| Acide salicylique | 0,2 % |
| Alcool cétylique | 10 % |
| Extrait de ruscus | 1 % |
| Conservateurs | qs |
| Microcapsules thermostabilisatrices | 10 % |
| Eau | qsp 100 % |

## Revendications

1. Composition topique comprenant, dans un support physiologiquement acceptable, l'association
- d'au moins un principe actif cosmétique ou pharmaceutique et
- de microcapsules renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, lesdites microcapsules ayant une enveloppe étanche, imperméable au composé cristallin à l'état fondu.

2. Composition selon la revendication 1, **caractérisée par le fait que** le point de fusion du composé cristallin encapsulé est compris entre 35 et 45 °C.

3. Composition selon l'une des revendications précédentes, **caractérisée par le fait que** le composé cristallin encapsulé présente une enthalpie de fusion comprise entre 100 et 300 kJ/kg, de préférence entre 150 et 280 kJ/kg.

4. Composition selon l'une des revendications précédentes, **caractérisée par le fait que** le composé cristallin encapsulé est choisi parmi
- les hydrocarbures aliphatiques contenant de 13 à 28 atomes de carbone,
- les hydrocarbures aromatiques,
- les acides gras saturés ou insaturés en C₉₋₂₄,
- les alcools gras saturés, linéaires ou ramifiés, en C₁₄₋₃₆,
- les esters des acides gras en C₁₀₋₂₂,
- les sels minéraux contenant une importante fraction d'eau de cristallisation,
- les triglycérides d'acides gras en C₁₂₋₁₈,
- les cires de silicone
- les dérivés de cire d'abeille, et
- les polymères thermofusibles cristallins.

5. Composition selon l'une quelconque des revendications précédentes, **caractérisée par le fait que** le composé cristallin encapsulé est un hydrocarbure aliphatique à chaîne linéaire contenant de 19 à 28 atomes de carbone, de préférence de 20 à 23 atomes de carbone.

6. Composition selon l'une quelconque des revendications précédentes, **caractérisée par le fait que** le matériau formant l'enveloppe étanche est choisi parmi les polyamides, les polyuréthannes, les polyurées, les polyesters, les polycyanoacrylates, les résines urée-formaldéhyde ou mélamine-formaldéhyde, les systèmes gélatine/gomme arabique et la silice.

7. Composition selon l'une quelconque des revendications précédentes, **caractérisée par le fait que** les microcapsules ont un diamètre moyen compris entre 0,01 et 100 micromètres, de préférence entre 0,05 et 50 micromètres.

8. Composition selon l'une quelconque des revendications précédentes, **caractérisée par le fait que** les microcapsules représentent de 0,1 à 95 % en poids, de préférence de 5 à 90 % en poids de la composition topique finale.

9. Composition selon l'une quelconque des revendications précédentes, **caractérisée par le fait que** le principe actif cosmétique ou pharmacologique est choisi parmi
- les agents anti-rides,
- les agents antibactériens et/ou antifongiques,
- les agents anti-acnéiques,
- les agents anti-radicaux libres et/ou détoxifiants,
- les agents kératolytiques,
- les accélérateurs de bronzage,
- les colorants naturels extraits de végétaux ou d'animaux,
- les actifs autobronzants,
- les liporégulateurs,
- les agents hydratants,
- les agents antipelliculaires,
- les agents anti-chute des cheveux.

10. Composition selon l'une quelconque des revendications précédentes, **caractérisée par le fait qu'**il s'agit d'un masque pour le visage ou les cheveux, d'un produit de massage, d'une composition amincissante, d'une crème hydratante, d'une crème anti-rides, d'une crème solaire, d'une composition de nettoyage, d'une lotion, d'un cataplasme, d'un produit relaxant et d'une crème dépilatoire.

11. Composition topique comprenant, dans un support physiologiquement acceptable, l'association
- d'au moins un principe actif pharmaceutique et
- de microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30°C,
en tant que médicament.

12. Procédé de traitement cosmétique comprenant les étapes consistant
- à chauffer une composition selon l'une quelconque des revendications 1 à 10 contenant au moins un principe actif cosmétique et des microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, jusqu'à une température supérieure au point de fusion desdites microcapsules,
- à appliquer la composition cosmétique sur la zone de la peau à traiter,
- à laisser la composition cosmétique refroidir au contact de la peau pendant un temps suffisant pour obtenir l'effet cosmétique recherché, et éventuellement,
- à éliminer la composition cosmétique.

13. Procédé de traitement cosmétique comprenant les étapes consistant
- à appliquer une composition cosmétique contenant au moins un principe actif cosmétique sur la zone de la peau à traiter,
- à chauffer une deuxième composition contenant, dans un support cosmétiquement acceptable, des microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, jusqu'à une température supérieure au point de fusion desdites microcapsules,
- à appliquer cette deuxième composition sur la zone de la peau à traiter, au dessus de la première composition cosmétique,
- à laisser les deux compositions au contact de la peau pendant un temps suffisant pour obtenir l'effet cosmétique recherché, et éventuellement
- à nettoyer la zone de la peau à traiter.

14. Procédé de traitement cosmétique comprenant les étapes consistant
- à appliquer sur la zone du corps ou du visage à traiter une composition cosmétique selon l'une quelconque des revendications 1 à 10 contenant au moins un principe actif cosmétique et des microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C,
- à masser la zone enduite de manière à augmenter localement la température jusqu'à une valeur suffisante pour faire fondre le composé cristallin,
- à laisser la composition cosmétique agir et refroidir au contact de la peau pendant un temps suffisant pour obtenir l'effet cosmétique recherché, et éventuellement,
- à éliminer la composition cosmétique.

15. Utilisation de microcapsules à enveloppe étanche renfermant au moins un composé cristallin présentant une enthalpie de fusion (ΔH_{f}), mesurée par analyse enthalpique différentielle, comprise entre 75 et 330 kJ/kg et un point de fusion supérieur ou égal à 30 °C, pour la fabrication d'un produit destiné à améliorer l'activité et/ou la pénétration cutanée d'un principe actif pharmaceutique appliqué simultanément ou préalablement.

16. Substrat solide insoluble imprégné avec une composition selon l'une quelconque des revendications 1 à 11.

17. Substrat solide insoluble selon la revendication 16, **caractérisé par le fait qu'**il s'agit d'un substrat textile non tissé à base de fibres d'origine naturelle ou synthétique.

18. Substrat solide insoluble selon l'une des revendications 16 ou 17, **caractérisé par le fait que** qu'il a une surface comprise entre 0,005 m2 et 0,1 m2, de préférence entre 0,01 m2 et 0,05 m2.

19. Substrat solide insoluble selon l'une des revendications 16 à 18, **caractérisé par le fait que** le taux d'imprégnation, c'est-à-dire la quantité de composition cosmétique ou pharmaceutique rapportée au poids du substrat solide, est compris entre 200 % et 1000 %, de préférence entre 250 % et 350 %.

20. Substrat solide insoluble selon l'une des revendications 16 à 19, **caractérisé par le fait qu'**il se présente sous la forme de lingettes rectangulaires ou de compresses rondes.

## Patentansprüche

1. Zusammensetzung zur topischen Anwendung, die in einem physiologisch akzeptablen Träger die folgende Kombination aus
- mindestens einem kosmetischen oder pharmazeutischen Wirkstoff und
- Mikrokapseln enthält, die mindestens eine kristalline Verbindung einschließen, die eine durch dynamische Differenzkalorimetrie bestimmte Schmelzenthalpie (ΔH_{f}) im Bereich von 75 bis 330 kJ/kg und einen Schmelzpunkt von 30 °C oder darüber aufweist, wobei die Mikrokapseln eine dichte Hülle besitzen, die für die kristalline Verbindung im geschmolzenen Zustand undurchlässig ist.

2. Zusammensetzung nach Anspruch 1, **dadurch gekennzeichnet, dass** der Schmelzpunkt der eingekapselten kristallinen Verbindung im Bereich von 35 bis 45 °C liegt.

3. Zusammensetzung nach einem der vorhergehenden Ansprüche, **dadurch gekennzeichnet, dass** die eingekapselte kristalline Verbindung eine Schmelzenthalpie von 100 bis 300 kJ/kg und vorzugsweise 150 bis 280 kJ/kg besitzt.

4. Zusammensetzung nach einem der vorhergehenden Ansprüche, **dadurch gekennzeichnet, dass** die eingekapselte kristalline Verbindung ausgewählt ist unter:
- aliphatischen Kohlenwasserstoffen mit 13 bis 28 Kohlenstoffatomen,
- aromatischen Kohlenwasserstoffen,
- gesättigten oder ungesättigten Fettsäuren mit 9 bis 24 Kohlenstoffatomen,
- gesättigten, geradkettigen oder verzweigten Fettalkoholen mit 14 bis 36 Kohlenstoffatomen,
- Fettsäureestern mit 10 bis 22 Kohlenstoffatomen,
- anorganischen Salzen, die einen hohen Anteil an Kristallwasser aufweisen,
- Triglyceriden von Fettsäuren mit 12 bis 18 Kohlenstoffatomen,
- Siliconwachsen,
- Derivaten von Bienenwachs, und
- in der Wärme schmelzbaren kristallinen Polymeren.

5. Zusammensetzung nach einem der vorhergehenden Ansprüche, **dadurch gekennzeichnet, dass** die eingekapselte kristalline Verbindung ein aliphatischer Kohlenwasserstoff mit gerader Kette ist, der 19 bis 28 Kohlenstoffatome und vorzugsweise 20 bis 23 Kohlenstoffatome enthält.

6. Zusammensetzung nach einem der vorhergehenden Ansprüche, **dadurch gekennzeichnet, dass** das Material, das die dichte Hülle bildet, unter den Polyamiden, Polyurethanen, Polyharnstoffen, Polyestern, Polycyanoacrylaten, Harnstoff Formaldehyd-Harzen oder Melanin-Formaldehyd-Harzen, Systemen Gelatine/Gummi arabicum und Kieselsäure ausgewählt ist.

7. Zusammensetzung nach einem der vorhergehenden Ansprüche, **dadurch gekennzeichnet, dass** die Mikrokapseln einen mittleren Durchmesser von 0,01 bis 100 µm und vorzugsweise 0,05 bis 50 µm aufweisen.

8. Zusammensetzung nach einem der vorhergehenden Ansprüche, **dadurch gekennzeichnet, dass** die Mikrokapseln 0,1 bis 95 Gew.% und vorzugsweise 5 bis 90 Gew.-% der fertigen Zusammensetzung zur topischen Anwendung ausmachen.

9. Zusammensetzung nach einem der vorhergehenden Ansprüche, **dadurch gekennzeichnet, dass** der kosmetische oder pharmazeutische Wirkstoff ausgewählt ist unter:
- Wirkstoffen gegen Falten,
- antibakteriellen und/oder fungiziden Wirkstoffen,
- Wirkstoffen gegen Akne,
- Radikalfängem für freie Radikale und/oder entgiftenden Wirkstoffen,
- Keratolytika,
- Bräunungsbeschleunigern,
- natürlichen, aus Pflanzen oder Tieren extrahierten Farbmitteln,
- Selbstbräunüngsmitteln,
- Liporegulatoren,
- Hydratisierungsmitteln,
- Antischuppenmitteln, und
- Wirkstoffen gegen Haarausfall.

10. Zusammensetzung nach einem der vorhergehenden Ansprüche, **dadurch gekennzeichnet, dass** es sich um eine Maske für das Gesicht oder die Haare, ein Produkt für die Massage, eine schlanker machende Zusammensetzung, eine hydratisierende Creme, eine Creme gegen Falten, eine Sonnenschutzcreme, eine Reinigungszusammensetzung, eine Lotion, ein Kataplasma, ein relaxierendes Produkt und eine Enthaarungscreme handelt.

11. Zusammensetzung zur topischen Anwendung, die in einem physiologisch akzeptablen Träger die Kombination aus
- mindestens einem pharmazeutischen Wirkstoff und
- Mikrokapseln mit dichter Hülle enthält, die mindestens eine kristalline Verbindung einschließen, die eine mit dynamischer Differenzkalorimetrie gemessene Schmelzenthalpie (ΔH_{f}) von 75 bis 330 kJ/kg und einen Schmelzpunkt von 30 °C oder darüber aufweist,
als Arzneimittel.

12. Verfahren zur kosmetischen Behandlung, das die folgenden Schritte umfasst:
- eine Zusammensetzung nach einem der Ansprüche 1 bis 10, die mindestens einen kosmetischen Wirkstoff und Mikrokapseln mit dichter Hülle enthält, die mindestens eine kristalline Verbindung einschließen, die eine mit dynamischer Differenzkalorimetrie gemessene Schmelzenthalpie (ΔH_{f}) von 75 bis 330 kJ/kg und einen Schmelzpunkt von 30 °C oder darüber aufweist, bis auf eine Temperatur über dem Schmelzpunkt der Mikrokapseln zu erwärmen,
- die kosmetische Zusammensetzung auf die zu behandelnde Hautzone aufzutragen,
- die kosmetische Zusammensetzung während einer Zeitspanne, die ausreichend ist, um die gewünschte kosmetische Wirkung zu erzielen, in Kontakt mit der Haut zu belassen, und gegebenenfalls
- die kosmetische Zusammensetzung zu entfernen.

13. Verfahren zur kosmetischen Behandlung, das die folgenden Schritte umfasst:
- eine kosmetische Zusammensetzung, die mindestens einen kosmetischen Wirkstoff enthält, auf die zu behandelnde Hautzone aufzutragen,
- eine zweite Zusammensetzung, die in einem kosmetisch akzeptablen Träger Mikrokapseln mit dichter Hülle enthält, die mindestens eine kristalline Verbindung einschließen, die eine mit dynamischer Differenzkalorimetrie gemessene Schmelzenthalpie (ΔH_{f}) von 75 bis 330 kJ/kg und einen Schmelzpunkt von 30 °C oder darüber aufweist, auf eine Temperatur über den Schmelzpunkt der Mikrokapseln zu erwärmen,
- diese zweite Zusammensetzung über der ersten kosmetischen Zusammensetzung auf die zu behandelnde Hautzone aufzutragen,
- die beiden Zusammensetzungen während einer Zeitspanne, die ausreichend ist, um die gewünschte kosmetische Wirkung zu erzielen, mit der Haut in Kontakt zu belassen, und gegebenenfalls
- die zu behandelnde Hautzone zu reinigen.

14. Verfahren zur kosmetischen Behandlung, das die folgenden Schritte umfasst:
- auf die zu behandelnde Zone des Körpers oder des Gesichts eine kosmetische Zusammensetzung nach einem der Ansprüche 1 bis 10 aufzutragen, die mindestens einen kosmetischen Wirkstoff und Mikrokapseln mit dichter Hülle enthält, die mindestens eine kristalline Verbindung einschließen, die eine mit dynamischer Differenzkalorimetrie gemessene Schmelzenthalpie (DH_{f}) von 75 bis 330 kJ/kg und einen Schmelzpunkt von 30 °C oder darüber aufweist,
- den beschichteten Bereich zu massieren, um die Temperatur lokal auf einen Wert zu erhöhen, der ausreichend ist, um die kristalline Verbindung zu schmelzen,
- die kosmetische Zusammensetzung einwirken zu lassen und im Kontakt mit der Haut während einer Zeitspanne, die ausreichend ist, um die gewünschte kosmetische Wirkung zu erhalten, abkühlen zu lassen, und gegebenenfalls
- die kosmetische Zusammensetzung zu entfernen.

15. Verwendung von Mikrokapseln mit dichter Hülle, die mindestens eine kristalline Verbindung enthalten, die eine mit dynamischer Differenzkalorimetrie gemessene Schmelzenthalpie (ΔH_{f}) im Bereich von 75 bis 330 kJ/kg und einen Schmelzpunkt von 30 °C oder darüber aufweist, zur Herstellung eines Produkts, das dazu vorgesehen ist, die Aktivität und/oder Hautpenetration eines gleichzeitig oder vorab aufgebrachten pharmazeutischen Wirkstoffs zu verbessern.

16. Festes unlösliches Substrat, das mit einer Zusammensetzung nach einem der Ansprüche 1 bis 11 imprägniert ist.

17. Festes unlösliches Substrat nach Anspruch 16, **dadurch gekennzeichnet, dass** es sich um einen textilen Vliesstoff auf der Basis von Fasern natürlicher oder synthetischer Herkunft handelt.

18. Festes unlösliches Substrat nach einem der Ansprüche 16 oder 17, **dadurch gekennzeichnet, dass** es eine Oberfläche im Bereich von 0,005 bis 0,1 m² und vorzugsweise 0,01 bis 0,05 m² besitzt.

19. Festes unlösliches Substrat nach einem der Ansprüche 16 bis 18, **dadurch gekennzeichnet, dass** der Imprägnierungsgrad, d. h. die Menge der kosmetischen oder pharmazeutischen Zusammensetzung, bezogen auf das Gewicht des festen Substrats, im Bereich von 200 bis 1000 % und vorzugsweise 250 bis 350 % liegt.

20. Festes unlösliches Substrat nach einem der Ansprüche 16 bis 19, **dadurch gekennzeichnet, dass** es in Form von rechteckigen Tüchern oder runden Kompressen vorliegt.

## Claims

1. Topical composition comprising, in a physiologically acceptable support, a combination
- of at least one cosmetic or pharmaceutical active principle and
- of microcapsules containing at least one crystalline compound with a heat of fusion (ΔH_{f}), measured by differential thermal analysis, of between 75 and 330 kJ/kg and a melting point of greater than or equal to 30°C, the said microcapsules having a leaktight envelope which is impervious to the molten crystalline compound.

2. Composition according to Claim 1, **characterized in that** the melting point of the encapsulated crystalline compound is between 35 and 45°C.

3. Composition according to one of the preceding claims, **characterized in that** the encapsulated crystalline compound has a heat of fusion of between 100 and 300 kJ/kg and preferably between 150 and 280 kJ/kg.

4. Composition according to one of the preceding claims, **characterized in that** the encapsulated crystalline compound is chosen from
- aliphatic hydrocarbons containing from 13 to 28 carbon atoms,
- aromatic hydrocarbons,
- saturated or unsaturated C₉₋₂₄ fatty acids,
- saturated, linear or branched C₁₄₋₃₆ fatty alcohols,
- C₁₀₋₂₂ fatty acid esters,
- mineral salts containing a large fraction of water of crystallization,
- C₁₂₋₁₈ fatty acid triglycerides,
- silicone waxes,
- beeswax derivatives, and
- crystalline hot-melt polymers.

5. Composition according to any one of the preceding claims, **characterized in that** the encapsulated crystalline compound is an aliphatic hydrocarbon with a linear chain containing from 19 to 28 carbon atoms and preferably from 20 to 23 carbon atoms.

6. Composition according to any one of the preceding claims, **characterized in that** the material forming the leaktight envelope is chosen from polyamides, polyurethanes, polyureas, polyesters, polycyanoacrylates, urea-formaldehyde or melamine-formaldehyde resins, gelatin/gum arabic systems and silica.

7. Composition according to any one of the preceding claims, **characterized in that** the microcapsules have a mean diameter of between 0.01 and 100 micrometres and preferably between 0.05 and 50 micrometres.

8. Composition according to any one of the preceding claims, **characterized in that** the microcapsules represent from 0.1% to 95% by weight and preferably from 5% to 90% by weight of the final topical composition.

9. Composition according to any one of the preceding claims, **characterized in that** the cosmetic or pharmacological active principle is chosen from
- anti-wrinkle agents,
- antibacterial and/or antifungal agents,
- antiacne agents,
- free-radical scavengers and/or detoxifying agents,
- keratolytic agents,
- tanning accelerators,
- natural colorants extracted from plants or animals,
- self-tanning active agents,
- liporegulators,
- moisturizers,
- antidandruff agents,
- agents for preventing hair loss.

10. Composition according to any one of the preceding claims, **characterized in that** it is a mask for the face or the hair, a massage product, a slimming composition, a moisturizing cream, an anti-wrinkle cream, an antisun cream, a cleansing composition, a lotion, a poultice, a relaxing product or a hair-removing cream.

11. Topical composition comprising, in a physiologically acceptable support, a combination
- of at least one pharmaceutical active principle, and
- of microcapsules with a leaktight envelope containing at least one crystalline compound with a heat of fusion (ΔH_{f}), measured by differential thermal analysis, of between 75 and 330 kJ/kg and a melting point of greater than or equal to 30°C, as a medicinal product.

12. Cosmetic treatment process comprising the steps consisting
- in heating a composition according to any one of Claims 1 to 10 containing at least one cosmetic active principle and microcapsules with a leaktight envelope containing at least one crystalline compound with a heat of fusion (ΔH_{f}), measured by differential thermal analysis, of between 75 and 330 kJ/kg and a melting point of greater than or equal to 30°C, up to a temperature above the melting point of the said microcapsules,
- in applying the cosmetic composition to the area of skin to be treated,
- in leaving the cosmetic composition to cool on contact with the skin for a time which is sufficient to obtain the desired cosmetic effect, and optionally
- in removing the cosmetic composition.

13. Cosmetic treatment process comprising the steps consisting
- in applying a cosmetic composition containing at least one cosmetic active principle to the area of skin to be treated,
- in heating a second composition containing, in a cosmetically acceptable support, microcapsules with a leaktight envelope containing at least one crystalline compound with a heat of fusion (ΔH_{f}), measured by differential thermal analysis, of between 75 and 330 kJ/kg and a melting point of greater than or equal to 30°C, up to a temperature above the melting point of the said microcapsules,
- in applying this second composition to the area of skin to be treated, over the first cosmetic composition,
- in leaving the two compositions in contact with the skin for a time which is sufficient to obtain the desired cosmetic effect, and optionally
- in cleaning the area of skin to be treated.

14. Cosmetic treatment process comprising the steps consisting
- in applying to the area of the body or the face to be treated a cosmetic composition according to any one of Claims 1 to 10 containing at least one cosmetic active principle and microcapsules with a leaktight envelope containing at least one crystalline compound with a heat of fusion (ΔH_{f}), measured by differential thermal analysis, of between 75 and 330 kJ/kg and a melting point of greater than or equal to 30°C,
- in massaging the coated area so as to increase the local temperature up to a value which is sufficient to melt the crystalline compound,
- in leaving the cosmetic composition to act and cool on contact with the skin for a time which is sufficient to obtain the desired cosmetic effect, and optionally
- in removing the cosmetic composition.

15. Use of microcapsules with a leaktight envelope containing at least one crystalline compound with a heat of fusion (ΔH_{f}), measured by differential thermal analysis, of between 75 and 330 kJ/kg and a melting point of greater than or equal to 30°C, for the manufacture of a product intended to improve the cutaneous activity and/or penetration of a pharmaceutical active principle applied simultaneously or beforehand.

16. Insoluble solid substrate impregnated with a composition according to any one of Claims 1 to 11.

17. Insoluble solid substrate according to Claim 16, **characterized in that** it is a nonwoven textile substrate made of fibres of natural or synthetic origin.

18. Insoluble solid substrate according to either of Claims 16 and 17, **characterized in that** it has a surface area of between 0.005 m² and 0.1 m² and preferably between 0.01 m² and 0.05 m².

19. Insoluble solid substrate according to one of Claims 16 to 18, **characterized in that** the degree of impregnation, that is to say the amount of cosmetic or pharmaceutical composition relative to the weight of the solid substrate, is between 200% and 1 000% and preferably between 250% and 350%.

20. Insoluble solid substrate according to one of Claims 16 to 19, **characterized in that** it is in the form of rectangular wipes or round compresses.
